# EUROPÄISCHE PATENTANMELDUNG

(11) **EP 3 461 457 A1**
(43) Veröffentlichungstag der Anmeldung: **03.04.2019**
(21) Anmeldenummer: 17193609.9
(22) Anmeldetag: 28.09.2017
(51) Int. Cl.: A61C 19/04, A61C 7/36, A61C 7/12, A61C 7/08, G07C 3/02, A61C 7/30

(54) **SENSOR UND SYSTEM ZUR ÜBERWACHUNG DER TRAGEDAUER VON KIEFERORTHOPÄDISCHEN GUMMIZÜGEN**

(71) Anmelder: Otmar Kronenberg AG, 6003 Luzern (CH)
(72) Erfinder: Kronenberg, Otmar, 6003 Luzern (CH)
(74) Vertreter: Koelliker, Robert

(57) **Zusammenfassung**

Es wird ein Sensor (1) zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3') beansprucht, um die Tragedauer von kieferorthopädischen Gummizügen (4) für Multibracket-Apparaturen (2, 3) und/oder Aligner (2', 3') zu überwachen, wobei der Sensor (1) ein von der Multibracket-Apparatur (2, 3) und den Brackets (23) unabhängiger Sensor ist und eine Gummizug-Halterung (16) und/oder Gummizug-Führung (17) umfasst.

Beansprucht wird auch ein System (5) zur Überwachung der Tragedauer von kieferorthopädischen Gummizügen, umfassend den Sensor (1), eine Multibracket-Apparatur oder einen Aligner für den Oberkiefer (2, 2') und den Unterkiefer (3, 3'), sowie einen Gummizug (4) und ein externes Lesegerät. Zudem wird auch ein Verfahren zum Überwachen der Tragedauer von kieferorthopädischen Gummizügen sowie die Verwendung des Sensors (1) und des Systems (5) beansprucht.

## Beschreibung

Die vorliegende Erfindung betrifft einen Sensor und ein System zur Überwachung der Tragedauer von kieferorthopädischen Gummizügen, ein Verfahren zum Überwachen der Tragedauer sowie die Verwendung des Sensors und des Systems.

Zahnfehlstellungen werden in der Regel mittels sogenannten Zahnspangen korrigiert. Einerseits gibt es abnehmbare Zahnspangen, d.h. solche Spangen, die der Patient auf einfache Art und Weise selber entfernen und wieder einsetzen kann, beispielsweise Aligner. Andererseits gibt es sogenannt festsitzende Zahnspangen, welche nur durch den Zahnarzt an den Zähnen angebracht und wieder entfernt werden können, sogenannte Multibracket-Apparaturen.

Zur Justierung der Position des Oberkiefers zum Unterkiefer kommen während einer Spangenkorrektur oft Gummizüge zum Einsatz, welche an festsitzenden Zahnspangen oder an Alignern befestigt werden. Dabei wird typischerweise am Ober- und am Unterkiefer je eine Multibracket-Apparatur und/oder ein Aligner angebracht. Die Multibracket-Apparaturen und Aligner weisen Vorrichtungen auf, an welchen der Gummizug auf einfache Art reversibel befestigt werden kann. Zur Justierung der Zahn- und Kieferstellung wird in der Regel der Gummizug an einem Bracket oder am Aligner im Oberkiefer und an einem Bracket oder am Aligner im Unterkiefer eingehängt. Durch den Gummizug entstehen intermaxilläre Kräfte, d.h. Kräfte zwischen dem Ober- und dem Unterkiefer, welche für die Justierung der Zahn- und Kieferstellung notwendig sind.

Solche Gummizüge können in aller Regel vom Patienten einfach entfernt und wieder angebracht werden. Um die gewünschte Justierung zu erzielen ist es zwingend, dass die Gummizüge gemäss den Vorgaben des Behandlers in der Regel über einen Zeitraum von mehr als 6 Stunden getragen werden.

Um den Heilungserfolg zu überprüfen muss der Patient regelmässig zur Kontrolle. Dabei kann es vorkommen, dass trotz Zahnspange mit Gummizug kein oder nur ein geringer Heilungsfortschritt beobachtet werden kann. In solchen Fällen weiss der Zahnarzt nicht, ob dies medizinisch begründet ist, oder ob der Patient die geforderte Mindesttragedauer nicht eingehalten hat.

Daher ist es die Aufgabe der vorliegenden Erfindung, die Tragedauer von Gummizügen bei kieferorthopädischen Behandlungen zu messen und zu dokumentieren, um objektive, und somit Patienten-unabhängige Angaben über die Tragedauer der Gummizüge zu erhalten.

Diese Aufgabe konnte überraschenderweise gelöst werden mit einem Sensor (1) zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3'), um die Tragedauer von kieferorthopädischen Gummizügen (4) für Multibracket-Apparaturen (2, 3) und/oder Aligner (2', 3') zu überwachen, wobei die Multibracket-Apparaturen eine Vielzahl von Brackets (23) aufweisen, umfassend einen Druck-, Zug- und/oder Scherkraftsensor (11) und einen Datenspeicher (12), wobei der Sensor (1)
- ein von der Multibracket-Apparatur (2, 3) und den Brackets (23) unabhängiger Sensor ist, sowie
- eine Befestigungsvorrichtung (15) zur Befestigung des Sensors (1) an einer Multibracket-Apparatur (2, 3) und/oder an einem Aligner (2', 3') aufweist, wobei der Sensor (1) anstelle der Befestigungsvorrichtung (15) auch an einem Aligner (2', 3') befestigt sein kann, und
- eine Gummizug-Halterung (16) und/oder Gummizug-Führung (17) umfasst.

Beansprucht wird auch ein System (5) zur Überwachung der Tragedauer von kieferorthopädischen Gummizügen für Multibracket-Apparaturen (2, 3) und/oder Aligner (2', 3'), umfassend einen erfindungsgemässen Sensor (1), eine Multibracket-Apparatur oder einen Aligner für den Oberkiefer (2, 2') und eine Multibracket-Apparatur oder einen Aligner für den Unterkiefer (3, 3'), sowie einen Gummizug (4) und ein externes Lesegerät, wobei die Multibracket-Apparaturen (2, 3) eine Vielzahl von Brackets (23) und je einen Bogendraht (24) umfassen.

Zudem wird auch ein Verfahren zum Überwachen der Tragedauer von kieferorthopädischen Gummizügen an Multibracket-Apparaturen (2, 3) und/oder Aligner (2', 3') mit dem erfindungsgemässen System (5) beansprucht, wobei
i) ein erfindungsgemässe Sensor (1) an einer Multibracket-Apparatur (2, 3) und/oder an einem Aligner (2', 3') befestigt wird,
ii) der Gummizug (4) von einer Multibracket-Apparatur (2, 3) und/oder von einem Aligner (2', 3') zur anderen Multibracket-Apparatur (2, 3) und/oder Aligner (2', 3') gespannt wird, wobei der Gummizug (4) an der Gummizug-Halterung (16) befestigt wird und/oder der Gummizug (4) in der Gummizug-Führung (17) geführt wird, wodurch durch den Gummizug (4) eine Kraft und/oder eine Scherkraft auf den Druck-, Zug- und/oder Scherkraftsensor (11) des Sensors (1) ausgeübt wird,
iii) mindestens die Zeitspanne der auf den Druck-, Zug- und/oder Scherkraftsensor (11) ausgeübten Kraft und/oder Scherkraft gemessen und auf dem Datenspeicher (12) gespeichert wird, und
iv) die im Datenspeicher (12) gespeicherten Daten unter Verwendung des Lesegeräts ausgelesen werden.

Beansprucht wird auch die Verwendung des erfindungsgemässen Sensors (1), des erfindungsgemässen Systems (5) und des erfindungsgemässen Verfahrens zum Überwachen der Tragedauer von kieferorthopädischen Gummizügen an Multibracket-Apparaturen (2, 3) und Alignern (2', 3') und/oder zur Messung der durch den Gummizug (4) ausgeübten Kraft, insbesondere der ausgeübten Kraft in Funktion der Tragedauer.

Der erfindungsgemässe Sensor (1), das erfindungsgemässe System (5), das erfindungsgemässe Verfahren und die erfindungsgemässe Verwendung weisen überraschenderweise eine Vielzahl von Vorteilen auf. So kann die Zeit, während welcher der Patient die Gummizüge trägt, d.h. die Tragedauer der Gummizüge durch den Patienten, auf einfache Art und Weise gemessen werden, und somit auch die Zeit, während welcher eine korrigierende Kraft auf die Zähne und Kiefer ausgeübt wird. Somit muss sich der Zahnarzt nicht mehr auf die Aussage des Patienten abstützen, sondern er hat ein neutrales Beurteilungsmittel.

Da der erfindungsgemässe Sensor (1) nicht in einem Bracket (23) integriert ist - und somit Bracket unabhängig ist - kann der Sensor (1) im Wesentlichen an jeder Multibracket-Apparatur (2, 3) und an jedem Aligner (2', 3') befestigt werden. Mit anderen Worten: Der erfindungsgemässe Sensor (1) kann auf einfache Art und Weise an jeder geeigneten Stelle jeder Multibracket-Apparatur (2, 3) oder an jedem Aligner (2', 3') aller Hersteller befestigt werden. Somit ist das erfindungsgemässe System (5) vorteilhafterweise nicht auf Multibracket-Apparaturen (2, 3) eines oder einiger wenigen Hersteller limitiert, sondern kann jegliche Multibracket-Apparatur (2, 3) umfassen.

Zudem kann der Sensor (1) auch grössere Dimensionen als ein einzelnes Bracket (23) aufweisen, wodurch ein Sensor (1) hergestellt werden kann, welcher robust ist und den geforderten Anforderungen, auch beispielsweise in Bezug auf die Grösse der Batterie und des Funkmoduls, in allen Belangen genügen kann. So ist beispielsweise die Grösse des Sensors (1) nicht auf eine Zahnoberfläche limitiert, wie dies bei Brackets (23) der Fall ist, sondern der Sensor (1) kann sich problemlos auch über zwei oder mehr Zahnoberflächen des gleichen Kiefers erstrecken.

Die WO-A-03/096922 beschreibt ein kieferorthopädisches Bracket zur Verankerung einer festsitzenden kieferorthopädischen Apparatur an einem Zahn. Das Bracket umfasst eine zu verankernde Bracketbasis und eine Bracketbefestigung, wobei zwischen der Bracketbasis und der Bracketbefestigung eine Sensoreinrichtung angeordnet ist. Damit werden die auf die Zähne angewendeten Kräfte, Drücke und/oder Drehmomente objektiv erfasst und gemessen. Dadurch kann die kieferorthopädische Apparatur so eingestellt werden, dass maximale Kräfte und Drehmomente auf die Zähne des Patienten wirken um einen optimalen Heilungserfolg zu erzielen. Somit wird ein Bracket mit integriertem Sensor beschrieben. Nicht erwähnt ist jedoch ein von einem Bracket unabhängigen Sensor, der auf jede Multibracket-Apparatur befestigt werden kann. Zudem wird auch die Messung der intermaxillären Kräfte mittels eines Gummizuges weder erwähnt noch vorgeschlagen.

Erfindungsgemäss wir unter dem Begriff Multibracket-Apparaturen (2, 3), auch festsitzende Zahnspange genannt, eine festsitzende kieferorthopädische Apparatur verstanden, welche im Wesentlichen aus einer Vielzahl von - in der Regel unterschiedlichen - Brackets (23) und einem Bogendraht (24) besteht. Die Brackets (23) werden durch den Zahnarzt an der Aussenseite einer Vielzahl von Zähnen mindestens eines Kiefers geklebt.

Anschliessend wird ein Bogendraht (24), auch Behandlungsbogen genannt, entlang den Brackets (23) geführt. Im Laufe der Behandlung wird der Bogendraht (24) mehrmals geändert, wobei der jeweils neu eingesetzte Bogendraht (24) eine zunehmende Dimension und Rigidität aufweist, wodurch die Zähne bewegt werden. Solche Multibracket-Apparaturen mit Brackets (23) und Bogendraht (24) sind dem Fachmann bekannt und im Handel erhältlich.

Unter Brackets (23) werden erfindungsgemäss alle Arten von Brackets verstanden, welche bei der Herstellung von Multibracket-Apparaturen Anwendung finden können. Die jeweilige Art und Form der Brackets (23) wird je nach Zahn und dessen zu korrigierende Stellung individuell ausgewählt. So können Brackets (23) auch Häkchen zum Befestigen von Gummizügen aufweisen. Erfindungsgemäss umfasst der Begriff Brackets (23) auch Tubes oder Bänder genannt. Die Brackets können aus Stahl, insbesondere Edelstahl, Keramik oder Kunststoff gefertigt sein. Geeignete Brackets sind dem Fachmann bekannt und im Handel erhältlich.

Als Aligner (2', 3') werden erfindungsgemäss durchsichtige Kunststoffschienen verstanden, welche für die jeweilige Zahnstellung individuell gefertigt werden. Die Aligner sind in der Regel durchsichtig und können von Aussenstehenden kaum wahrgenommen werden. Bei Bedarf ist es in aller Regel möglich, am Aligner eine Gummizug-Halterung zu befestigen, oder bei der Herstellung des Aligners eine solche im Aligner zu integrieren. Hierzu kann beispielsweise eine Kerbe im Aligner (2', 3') angebracht werden. Alternativ kann auch eine Befestigungsmöglichkeit, beispielsweise ein Knöpfchen oder Button, direkt auf den Zahn geklebt werden, wobei der Aligner (3') an dieser Stelle eine Aussparung aufweist. Geeignete Befestigungsmöglichkeiten wie Knöpfchen oder Buttons sind dem Fachmann bekannt und im Handel erhältlich. Im Gegensatz zur festsitzenden Multibracket-Apparatur können Aligner vom Patienten selber herausgenommen werden, d.h. sie sind abnehmbar.

Unter dem Begriff Gummizug (4) wird erfindungsgemäss jedes geeignete Mittel verstanden, welches Zahnärzte bei Multibracket-Apparaturen (2, 3) resp. Alignern (2', 3') verwenden, um eine Kraft auf den Oberkiefer und/oder Unterkiefer auszuüben. Somit kann der Gummizug (4) auch aus einem anderen Material gefertigt sein als Gummi. Der Gummizug (4) ist typischerweise ein Gummiband, welches sich unter Kraftaufwand reversibel dehnen lässt. Besonders bevorzugt liegt der Gummizug (4) in Form von Gummiringen vor, d.h. in Form eines kurzen Stücks Gummi in Ringform. Mit dem Gummizug (4) wird die Multibracket-Apparatur (2) resp. der Aligner (2') des Oberkiefers mit der Multibracket-Apparatur (3) resp. der Aligner (3') des Unterkiefers verbunden. Dabei wird der Gummizug (4) bevorzugt an einem Bracket (23), am Bogendraht (24), an einem Zahn, an einem im Aligner (2', 3') integrierten Häkchen und/oder am Sensor (1), insbesondere an der Gummizug-Halterung (16) des Sensors (1), befestigt.

### Der Sensor (1)

Der erfindungsgemässe Sensor (1) eignet sich insbesondere zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3'), um die Tragedauer von kieferorthopädischen Gummizügen (4) für festsitzende Multibracket-Apparaturen (2, 3) und/oder abnehmbare Aligner (2', 3') zu überwachen, d.h. zu messen und zu speichern. Zusätzlich kann der Sensor (1) beispielsweise auch zur Messung der durch den Gummizug (4) ausgeübten Kraft - in Funktion der Tragedauer - eingesetzt werden. Pro Patient kann ein oder mehr als ein Sensor (1) gleichzeitig verwendet werden.

Der erfindungsgemässe Sensor (1) ist ein von der Multibracket-Apparatur (2, 3) und den Brackets (23) unabhängiger Sensor. Der Sensor (1) umfasst einen Druck-, Zug- und/oder Scherkraftsensor (11) und einen Datenspeicher (12), eine Befestigungsvorrichtung (15) zur Befestigung des Sensors (1) an einer Multibracket-Apparatur (2, 3) und/oder an einem Aligner (2', 3'), wobei der Sensor (1) anstelle der Befestigungsvorrichtung (15) auch an einem Aligner (2', 3') befestigt, insbesondere geklebt und/oder in den Aligner integriert, sein kann, und eine Gummizug-Halterung (16) und/oder Gummizug-Führung (17). Die Gummizug-Halterung (16) umfasst typischerweise mindestens eine Vorrichtung, beispielsweise ein Häkchen, zum Befestigen von Gummizügen.

Der Sensor (1) umfasst zudem in einer bevorzugten Ausführungsform ein Gehäuse (13), eine Stromversorgung (14), insbesondere eine Batterie, und/oder eine Datenübertragungsvorrichtung (18), insbesondere einen Funksender und/oder eine Steckverbindung, um die gemessenen und im Sensor (1) - insbesondere im Datenspeicher (12) - gespeicherten Daten auszulesen, d.h. zu einem anderen Medium, wie beispielsweise zu einem Computer, zu übertragen. Das Auslesen der Daten kann regelmässig - beispielsweise täglich - beispielsweise durch den Patienten und/oder automatisiert mittels Funkübermittlung, erfolgen. Oft ist es jedoch bevorzugt, dass das Auslesen bei der periodischen Kontrolle beim Zahnarzt erfolgt. Das Auslesen kann beispielsweise mittels Funk, insbesondere mittels NFC, RFID und/oder Bluetooth, und/oder mittels Steckverbindung zu einem externen Lesegerät übertragen werden. Dazu kann der Sensor (1) an der Multibracket-Apparatur (2, 3) resp. am Aligner (2', 3') befestigt bleiben, oder er kann zum Auslesen entfernt werden und gegebenenfalls anschliessend wieder an der Multibracket-Apparatur (2, 3) resp. am Aligner (2', 3') fixiert werden.

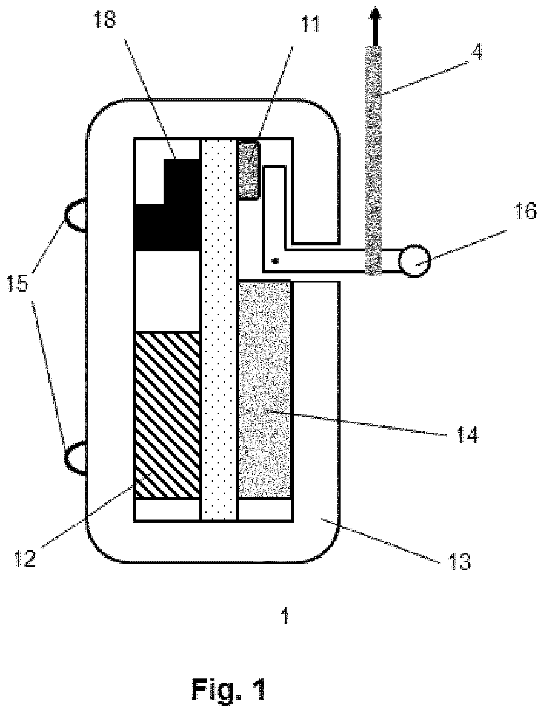

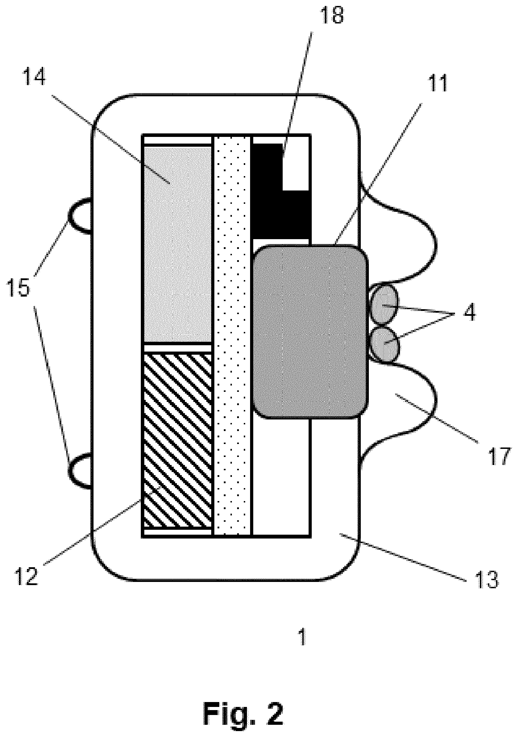

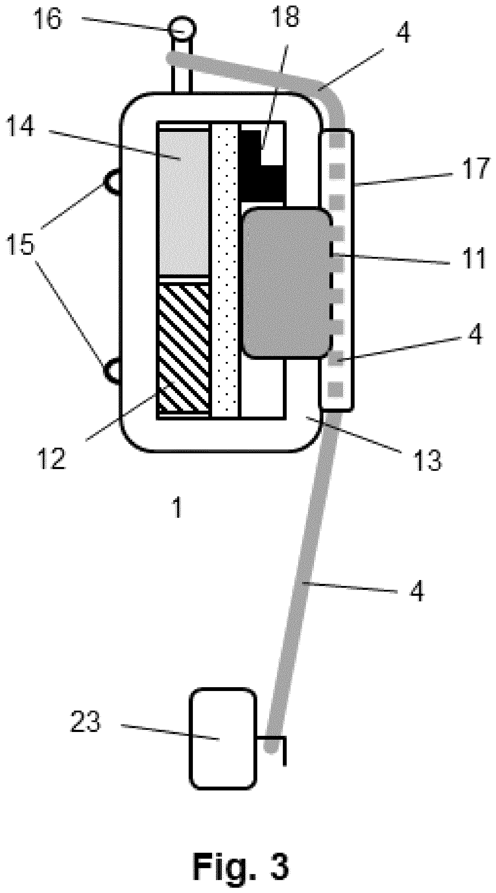

In einer anderen bevorzugten Ausführungsform des Sensors (1) kann ein Gummizug (4) an der Gummizug-Halterung (16) des Sensors (1) reversibel befestigt werden um die Gummizug-Halterung (16) mit dem Gummizug (4) mit einem Bracket (23), Bogendraht (24) oder Aligner (2', 3') zu verbinden. Dabei übt die Gummizug-Halterung (16) bei gespanntem Gummizug (4) gegebenenfalls - insbesondere bevorzugt - eine Kraft auf den Druck-, Zug- und/oder Scherkraftsensor (11) des Sensors (1) aus, wobei im Sensor (1) zumindest die Zeiten, während derer diese Kraft ausgeübt wurde, gemessen und gespeichert werden. Eine nicht-limitierende Ausführungsform ist in Fig. 1 dargestellt. Alternativ - oder in Kombination - kann ein Gummizug (4) in der Gummizug-Führung (17) des Sensors (1) geführt werden, wodurch eine Kraft auf den Druck-, Zug- und/oder Scherkraftsensor (11) ausgeübt wird, wenn der Gummizug (4) von einem Bracket (23) der Multibracket-Apparatur des Oberkiefers (2) oder vom Aligner des Oberkiefer (2') zu einem Bracket (23) der Multibracket-Apparatur des Unterkiefers (3) oder zum Aligner des Unterkiefers (3') angeordnet ist. Eine solche beispielhafte Anordnung ist in Fig. 2 dargestellt. Dabei kann gegebenenfalls, insbesondere bevorzugt, der Gummizug (4) anstelle an einem Bracket (23) oder Aligner (2', 3') auch an der Gummizug-Halterung (16) des Sensors (1) befestigt sein, wie in Fig. 3 gezeigt.

In einer bevorzugten Ausführungsform umfasst die Befestigungsvorrichtung (15) des Sensors (1) eine Öse, Klemme, Schraube, ein Draht, Gummizug, Steckverbindung, Elastik und/oder Klebefläche, anhand welcher der Sensor (1) im Wesentlichen an jeder beliebigen Multibracket-Apparatur (2, 3) und an jeden beliebigen Aligner (2', 3') befestigt werden. Dem Fachmann sind solche Befestigungsmittel und Befestigungsarten bekannt und er kann damit den erfindungsgemässen Sensor (1) an der Multibracket-Apparatur (2, 3) und am Aligner (2', 3') korrekt und genügend befestigen.

### Das System (5)

Das erfindungsgemässe System (5) ist insbesondere zur Überwachung, d.h. zum Messen und Speichern, der Tragedauer von kieferorthopädischen Gummizügen für Multibracket-Apparaturen (2, 3) und/oder Aligner (2', 3') geeignet. Überraschenderweise können im erfindungsgemässen System Brackets, Multibracket-Apparaturen, Aligner und Gummizüge von beliebigen Herstellern eingesetzt werden, sofern sie sich für kieferorthopädische Anwendungen eignen. Das System (5) umfassend den Sensor (1) kann zudem auch zur Messung der durch den Gummizug (4) ausgeübten Kraft - in Funktion der Tragedauer - eingesetzt werden.

Das System (5) umfasst einen erfindungsgemässen Sensor (1), eine Multibracket-Apparatur oder einen Aligner für den Oberkiefer (2, 2') und eine Multibracket-Apparatur oder einen Aligner für den Unterkiefer (3, 3'), sowie einen Gummizug (4) und ein externes Lesegerät, wobei die Multibracket-Apparaturen (2, 3) eine Vielzahl von Brackets (23) und je einen Bogendraht (24) umfassen. Dabei kann pro Patient ein Sensor (1), welcher am Ober- oder Unterkiefer des Patienten befestigt wird, genügen. Es ist aber auch möglich, dass beim Patienten zwei oder mehr Sensoren (1) eingesetzt werden, typischerweise pro eingesetzten Gummizug (4) ein Sensor (1). Bei Bedarf ist es auch möglich, sowohl am Oberkiefer wie auch am Unterkiefer einen Sensor (1) zu befestigen. Dabei umfasst vorteilhafterweise mindestens ein Bracket (23) der Multibracket-Apparatur oder des Aligners für den Oberkiefer (2, 2') oder der Multibracket-Apparatur oder des Aligners für den Unterkiefer (3, 3') eine Anordnung - insbesondere ein Häkchen, zum Befestigen von Gummizügen.

Im System (5) kann der Sensor (1) mittels der Befestigungsvorrichtung (15), welche mit dem Sensor (1) typischerweise starr verbunden ist, an einem oder mehreren Brackets (23) jeder beliebigen Multibracket-Apparatur (2, 3) sowie an jeden beliebigen Aligner (2', 3') befestigt werden. Es ist auch möglich, dass der Sensor (1) zusätzlich - oder nur - am Bogendraht (24) befestigt wird. Alternativ - oder in Kombination - kann er auch an einem oder mehreren Zähnen befestigt werden.

In einer bevorzugten Ausführungsform des Systems (5) kann die Befestigungsvorrichtung (15) des Sensors (1) mit mindestens einem Bracket (23) und/oder dem Bogendraht (24) einer Multibracket-Apparatur (2, 3), und/oder einem Aligner (2', 3'), mit einem Befestigungsmittel verbunden werden.

Geeignete - und oft auch bevorzugte - Befestigungsmittel zur Befestigung der Befestigungsvorrichtung (15) des Sensors (1) an einer Multibracket-Apparatur (2, 3) und/oder an einem Aligner (2', 3') umfassen einen Draht, eine Schraube, eine Klemme, einen Gummizug, einen Elastik, einen Kunststoff und/oder einen Klebstoff oder Klebstoffkombination. Dabei werden typischerweise die Befestigungsmittel mit der Befestigungsvorrichtung (15) des Sensors (1) abgestimmt, um eine geeignete Befestigung zu erhalten. Der Fachmann weiss, welche Befestigungsmittel für die spezifische Anwendung geeignet sind und welche Befestigungsmittel er mit welcher Befestigungsvorrichtung (15) optimal kombinieren kann. Wird zudem der Sensor (1) an einen Aligner befestigt, ist es auch möglich, den Sensor (1) direkt während der Herstellung des Aligners (2', 3') mit dem Sensor zu verbinden.

In einer bevorzugten Ausführungsform des erfindungsgemässen Systems (5) ist der erfindungsgemässe Sensor (1), wenn er an einer Multibracket-Apparatur (2, 3) befestigt ist, i) zwischen zwei Brackets (23) der gleichen Multibracket-Apparatur (2, 3) angeordnet, und/oder ii) erstreckt er sich über mindestens ein, bevorzugt über mindestens zwei, Brackets (23) der gleichen Multibracket-Apparatur (2, 3).

In einer anderen bevorzugten Ausführungsform des erfindungsgemässen Systems (5) ist der erfindungsgemässe Sensor (1), wenn er an einem Aligner (2', 3') befestigt ist, i) im seitlichen Aussenbereich des Aligners (2', 3') angeordnet, und/oder ii) erstreckt er sich über mindestens einen, bevorzugt über mindestens zwei, Zahnkronen des gleichen Aligners (2', 3').

### Das Verfahren

Das erfindungsgemässe Verfahren eignet sich besonders zum Überwachen, d.h. zum Messen und Speichern, der Tragedauer von kieferorthopädischen Gummizügen an Multibracket-Apparaturen (2, 3) und/oder Aligner (2', 3') mit dem erfindungsgemässen System (5). Zusätzlich kann das Verfahren beispielsweise auch zur Messung der durch den Gummizug (4) ausgeübten Kraft - in Funktion der Tragedauer - eingesetzt werden.

In einem ersten Schritt i) des erfindungsgemässen Verfahrens wird der erfindungsgemässe Sensor (1) - wie vorgängig beschrieben - an einer Multibracket-Apparatur (2, 3) und/oder an einem Aligner (2', 3'), typischerweise mit einem Befestigungsmittel mittels der Befestigungsvorrichtung (15), befestigt. Dabei erfolgt die Befestigung in des Sensors (1) an die Multibracket-Apparatur (2, 3) in der Regel nachdem die Multibracket-Apparatur an den Zähnen befestigt wurde. Die Befestigung des Sensors (1) an den Aligner (2', 3') kann während oder nach der Herstellung des Aligners (2', 3') erfolgen. Wird der Sensor (1) nach der Herstellung des Aligners (2', 3') am Aligner (2', 3') befestigt, kann die Befestigung des Sensors (1) an den Aligner (2', 3') ausserhalb des Mundes oder direkt im Mund erfolgen. Dieser Schritt erfolgt in aller Regel beim Zahnarzt.

In einem zweiten Schritt ii) des erfindungsgemässen Verfahrens wird der Gummizug (4) von einer Multibracket-Apparatur (2, 3) und/oder von einem Aligner (2', 3') zur anderen Multibracket-Apparatur (2, 3) und/oder Aligner (2', 3') gespannt, wobei der Gummizug (4) an der Gummizug-Halterung (16) des Sensors (1) befestigt und/oder der Gummizug (4) in der Gummizug-Führung (17) des Sensors (1) geführt wird, wodurch durch den Gummizug (4) eine Kraft und/oder eine Scherkraft auf den Druck-, Zug- und/oder Scherkraftsensor (11) des Sensors (1) ausgeübt wird. Dieser Schritt erfolgt zunächst direkt im Anschluss an Schritt i) beim Zahnarzt und dann regelmässig, d.h. typischerweise mehrmals täglich, durch den Patienten selber.

In einem weiteren Schritt iii) des erfindungsgemässen Verfahrens wird mindestens die Zeitspanne der auf den Druck-, Zug- und/oder Scherkraftsensor (11) ausgeübten Kraft und/oder Scherkraft gemessen, d.h. die Zeitspanne, während welcher die durch den Gummizug (4) erzeugte Kraft und/oder Scherkraft auf den Druck-, Zug- und/oder Scherkraftsensor (11) ausgeübt wird. Dabei wird die gemessene Zeitspanne auf dem Datenspeicher (12) des Sensors (1) gespeichert. Dieser Schritt erfolgt immer dann, wenn der Gummizug (4) an der Multibracket-Apparatur (2, 3) und/oder am Aligner (2', 3') befestigt ist.

In einem weiteren Schritt vi) des erfindungsgemässen Verfahrens werden die im Datenspeicher (12) des Sensors (1) gespeicherten Daten unter Verwendung des Lesegeräts ausgelesen werden. Dabei kann als Lesegerät ein spezifisches, beispielsweise für diesen Verfahrensschritt optimiertes Lesegerät, zur Anwendung kommen. Alternativ kann als Lesegerät auch ein Computer und/oder Mobiltelefon wie Smartphone dienen, welcher die Daten vom Datenspeicher (12) des Sensors (1) auslesen kann. Dabei erfolgt das Auslesen der Daten typischerweise mit üblichen Methoden, die dem Fachmann bekannt sind. Dieser Schritt erfolgt in aller Regel bei der Kontrolle und beim Nachjustieren beim Zahnarzt. Alternativ - oder zusätzlich - kann das Auslesen der Daten auch in kürzeren Zeitabständen, beispielsweise täglich, erfolgen. Dabei werden die Daten bevorzugt per Funk, und gegebenenfalls mittels Internet, zum Lesegerät gesendet. Das Lesegerät kann sich beispielsweise in der Zahnarzt Praxis befinden und/oder an einem anderen Ort, beispielsweise an einem zentralen Ort, von welchem aus die gesammelten Daten zu den einzelnen Zahnarzt Praxen weitergeleitet werden.

In einer bevorzugten Ausführungsform des erfindungsgemässen Verfahren werden die im Datenspeicher (12) des Sensors (1) gespeicherten Daten mittels Funk, insbesondere mittels NFC, RFID und/oder Bluetooth, und/oder mittels Steckverbindung, d.h. mittels Kabelverbindung, zum externen Lesegerät übertragen und gegebenenfalls gespeichert und/oder ausgedruckt.

### Die Verwendung

Der erfindungsgemässe Sensor (1), das erfindungsgemässe Systems (5) und das erfindungsgemässe Verfahrens eigenen sich zur Verwendung mit kieferorthopädischen Apparaturen, insbesondere Multibracket-Apparaturen (2, 3) und Alignern (2', 3') für Oberkiefer und Unterkiefer.

Eine besonders bevorzugte Verwendung umfasst das Überwachen - und somit das Messen und Speichern - der Tragedauer von kieferorthopädischen Gummizügen an Multibracket-Apparaturen und Alignern.

Der Sensor (1), das Systems (5) und das Verfahrens können aber auch - insbesondere zusätzlich zum Überwachen der Tragedauer - verwendet werden zur Messung der durch den Gummizug (4) ausgeübten Kraft, insbesondere der ausgeübten Kraft in Funktion der Tragedauer.

Es werden folgende Bezugszeichen verwendet:
- 1: Sensor
- 2: Multibracket-Apparatur für den Oberkiefer
- 3: Multibracket-Apparatur für den Unterkiefer
- 2': Aligner für den Oberkiefer
- 3': Aligner für den Unterkiefer
- 4: Gummizug
- 5: System
- 11: Druck-, Zug- und/oder Scherkraftsensor
- 12: Datenspeicher
- 13: Gehäuse
- 14: Stromversorgung
- 15: Befestigungsvorrichtung
- 16: Gummizug-Halterung
- 17: Gummizug-Führung
- 18: Datenübertragungsvorrichtung
- 23: Vielzahl von Brackets
- 24: Bogendraht

Im Folgenden werden nicht-limitierende, bevorzugte Ausführungsformen des erfindungsgemässen Sensors (1) und des erfindungsgemässen System (5) anhand der nachfolgenden Zeichnungen beschrieben. Diese sind nicht einschränkend auszulegen und werden als Bestandteil der Beschreibung verstanden:
- Fig. 1: zeigt beispielhaft eine nicht-limitierende Ausführungsform des erfindungsgemässen Sensors (1). In der Mitte des Gehäuses (13) ist eine Leiterplatte (schwach gemustert dargestellt) angeordnet, an welcher beispielhaft der Datenspeicher (12), als Stromversorgung (14) eine Batterie, als Datenübertragungsvorrichtung (18) ein Transmitter sowie einen Druck-, Zug- und/oder Scherkraftsensor (11). Der Datenspeicher (12), die Stromversorgung (14), Datenübertragungsvorrichtung (18) und der Druck-, Zug- und/oder Scherkraftsensor (11) sind über die Leiterplatte miteinander so verbunden, dass die gewünschten Informationen ausgetauscht werden können und dass die Stromversorgung der einzelnen Komponenten gewährleistet ist.
An der Gummizug-Halterung (16) des Sensors (1) ist ein Gummizug (4) angebracht. Dieser erstreckt sich nun von der einen Multibracket-Apparatur (2, 3), an welcher der Sensor (1) befestigt ist, in Richtung - mit dem Pfeil angedeutet - eines Häkchens eines Brackets (23) der anderen Multibracket-Apparatur (2, 3). Wird nun der Gummizug korrekterweise zwischen den beiden Multibracket-Apparaturen (2, 3) gespannt, wird auf die Gummizug-Halterung (16) des Sensors (1) ein Zug ausgeübt. Durch die L-förmige Anordnung der Gummizug-Halterung (16) mit Drehpunkt beim Ecken der Gummizug-Halterung (16) entsteht nun ein Druck auf den Sensor (11). Dieser misst zumindest die Zeitdauer, während welcher ein Druck ausgeübt wird und speichert die gemessenen Informationen auf dem Datenspeicher (12).
An der Aussenseite des Gehäuses (13), gegenüber der Gummizug-Halterung (16) sind Ösen als Befestigungsvorrichtung (15) des Sensor (1) angeordnet, mit welcher der Sensor (1) an der Multibracket-Apparatur (2, 3) fixiert werden kann.
- Fig. 2: zeigt beispielhaft eine weitere, nicht-limitierende Ausführungsform des erfindungsgemässen Sensors (1). Innerhalb des Gehäuses (13) ist der Datenspeicher (12), die Stromversorgung (14), die Daten-übertragungsvorrichtung (18) sowie der Druck-, Zug- und/oder Scherkraftsensor (11) angeordnet. Diese sind über die Leiterplatte (schwach gemustert) so miteinander verbunden, dass die gewünschten Informationen ausgetauscht werden können und dass die Stromversorgung der einzelnen Komponenten gewährleistet ist. An einer Aussenseite des Gehäuses (13) sind Ösen als Befestigungsvorrichtung (15) des Sensor (1) angeordnet, mit welcher der Sensor (1) an der Multibracket-Apparatur (2, 3) fixiert werden kann.
Auf der Seite des Gehäuses (13), die der Befestigungsvorrichtung (15) gegenüber liegt, ist - beispielhaft und im Querschnitt gezeigt-eine Gummizug-Führung (17) des Sensors (1) angeordnet, in welcher der Gummizug (4) liegt und Druck auf den Druck-, Zug- und/oder Scherkraftsensor (11) ausübt. Um die Empfindlichkeit des Sensors (11) zu erhöhen, reicht dieser beispielhaft etwas über die Abmessungen des Gehäuses (13) hinaus, sodass der Gummizug (4) einen etwas erhöhten Druck auf den Sensor (11) ausüben kann. Dazu ist der Gummizug (4) typischerweise an je einem Bracket (23), insbesondere an einem Häkchen eines Brackets (23), der Multibracket-Apparatur für den Oberkiefer (2) und der Multibracket-Apparatur für Unterkiefer (3) befestigt (nicht dargestellt).
- Fig. 3: zeigt beispielhaft eine andere, nicht-limitierende Ausführungsform des erfindungsgemässen Sensors (1). Analog der in Fig. 2 gezeigten Darstellung wird der Gummizug (4) durch die Gummizug-Führung (17) geführt, wobei jedoch der Gummizug (4) an der Gummizug-Halterung (16) des Sensors (1) befestigt ist und innerhalb der Gummizug-Führung (17) zu einem Bracket (23) der anderen Multibracket-Apparatur geführt wird, wo der Gummizug (4) an einem Häkchen befestigt ist.
Der Druck-, Zug- und/oder Scherkraftsensor (11) reicht wie in Fig. 2 beispielhaft etwas über die Abmessungen des Gehäuses (13) hinaus, sodass der Gummizug (4) einen etwas erhöhten Druck auf den Sensor (11) ausüben kann.

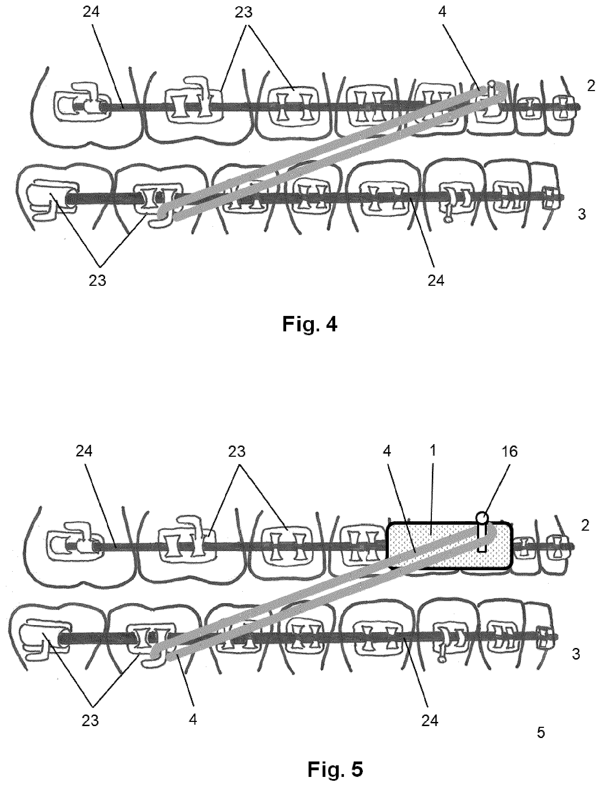

- Fig. 4: zeigt eine heute üblicherweise verwendete Multibracket-Apparatur für den Oberkiefer (2) und eine Multibracket-Apparatur für den Unterkiefer (3) mit jeweils einer Vielzahl von unterschiedlichen Brackets (23) ohne den erfindungsgemässen Sensor (1) und stellt somit den Stand der Technik dar. Einige der Brackets (23), die mit dem Bogendraht (24) miteinander verbunden sind, weisen ein Häkchen auf, an welchen beispielsweise ein Gummizug (4) befestigt werden kann. Vor dem Essen und der Zahnreinigung wird vorteilhafterweise der Gummizug (4) entfernt. Dabei ist es wichtig, dass nach dem Essen und nach der Zahnreinigung der Gummizug (4) wieder an den Häkchen der Brackets (23) befestigt wird, um die zur erfolgreichen Therapie notwendigen Kräfte zu erzeugen. Mit diesem heutzutage vielfach eingesetzten System kann jedoch nicht überprüft werden, ob die Gummizüge auch tatsächlich getragen werden.
- Fig. 5: zeigt das erfindungsgemässe System (5) umfassend den erfindungsgemässen Sensor (1), welcher beispielhaft an der Multibracket-Apparatur für den Oberkiefer (2), und somit an den Brackets (23) und/oder dem Bogendraht (24), befestigt ist. Der Sensor (1) erstreckt sich beispielhaft entlang von etwa zwei Zähnen und überdeckt dementsprechend auch zwei Brackets (23). Der Sensor (1) umfasst eine Gummizug-Halterung (16), an welcher der Gummizug (4) befestigt ist. Der Gummizug (4) führt weiter zur Multibracket-Apparatur des Unterkiefers (3), wo er an einem Häkchen eines Brackets (23) angebracht ist. Der Sensor (1) kann natürlich auch an der Multibracket-Apparatur für den Unterkiefer (3) angebracht werden. Bei gestrecktem Gummizug (4) wird dann durch die Gummizug-Halterung (16) ein Druck auf den im Sensor (1) angeordneten Druck-, Zug- und/oder Scherkraftsensor (11) ausgeübt. Dabei wird im Sensor (1) zumindest die Zeit gemessen und im Datenspeicher (12) des Sensors (1) gespeichert. Bei der nächsten Kontrolle beim Zahnarzt können dann die im Datenspeicher (12) gespeicherten Daten über die im Sensor (1) typischerweise integrierte Datenübertragungsvorrichtung (18) mittels einem Lesegerät (nicht dargestellt) ausgelesen und anschliessend ausgewertet werden.

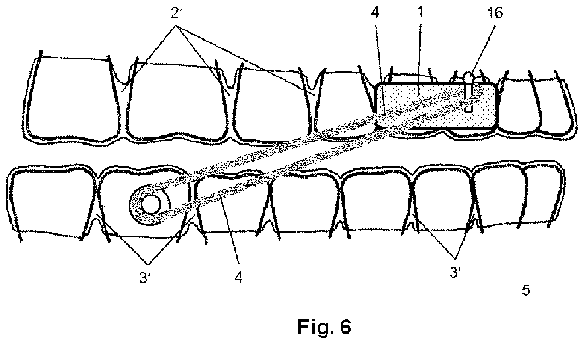

- Fig. 6: zeigt das erfindungsgemässe System (5) umfassend einen Aligner für den Oberkiefer (2') und einen Aligner für den Unterkiefer (3'). Die Aligner sind um die Zähne angeordnet, durch das Zahnfleisch begrenzt und mit einer dünneren Linie dargestellt. Der erfindungsgemässe Sensor (1) ist beispielhaft am Aligner für den Oberkiefer (2') angebracht und erstreckt sich entlang von etwa zwei Zähnen. Der Sensor (1) umfasst eine Gummizug-Halterung (16), an welcher der Gummizug (4) befestigt ist. Der Gummizug (4) führt weiter zum Aligner des Unterkiefers (3'), wo er in dieser Darstellung an einem runden Knöpfchen oder Button befestigt ist. Solche Knöpfchen oder Buttons sind dem Fachmann bekannt und werden typischerweise direkt auf den Zahn geklebt, wobei der Aligner (3') an dieser Stelle eine Aussparung aufweist. Alternativ kann der Gummizug beispielsweise an einer im Aligner (3') angebrachten Kerbe befestigt werden. Der Sensor (1) kann natürlich auch am Aligner für den Unterkiefer (3') und das Knöpfchen oder die Aussparung am Aligner für den Oberkiefer (2') angebracht werden. Bei gestrecktem Gummizug (4) wird dann durch die Gummizug-Halterung (16) ein Druck auf den im Sensor (1) angeordneten Druck-, Zug- und/oder Scherkraftsensor (11) ausgeübt. Dabei wird im Sensor (1) zumindest die Zeit gemessen und im Datenspeicher (12) des Sensors (1) gespeichert. Periodisch, beispielsweise bei der nächsten Kontrolle beim Zahnarzt, können dann die im Datenspeicher (12) gespeicherten Daten über die im Sensor (1) typischerweise integrierte Datenübertragungsvorrichtung (18) mittels einem Lesegerät (nicht dargestellt) ausgelesen und anschliessend ausgewertet werden.

## Patentansprüche

1. Sensor (1) zur Befestigung an einer Multibracket-Apparatur für den Oberkiefer (2), an einer Multibracket-Apparatur für den Unterkiefer (3), an einem Aligner für den Oberkiefer (2') und/oder an einem Aligner für den Unterkiefer (3'), um die Tragedauer von kieferorthopädischen Gummizügen (4) für Multibracket-Apparaturen (2, 3) und/oder Aligner (2', 3') zu überwachen, wobei die Multibracket-Apparaturen eine Vielzahl von Brackets (23) aufweisen, umfassend einen Druck-, Zug- und/oder Scherkraftsensor (11) und einen Datenspeicher (12), **dadurch gekennzeichnet, dass** der Sensor (1)
- ein von der Multibracket-Apparatur (2, 3) und den Brackets (23) unabhängiger Sensor ist, sowie
- eine Befestigungsvorrichtung (15) zur Befestigung des Sensors (1) an einer Multibracket-Apparatur (2, 3) und/oder an einem Aligner (2', 3') aufweist, wobei der Sensor (1) anstelle der Befestigungsvorrichtung (15) auch an einem Aligner (2', 3') befestigt sein kann, und
- eine Gummizug-Halterung (16) und/oder Gummizug-Führung (17) umfasst.

2. Sensor (1) nach Anspruch 1, **dadurch gekennzeichnet, dass** der Sensor (1) zudem ein Gehäuse (13), eine Stromversorgung (14) und/oder eine Datenübertragungsvorrichtung (18), insbesondere einen Funksender und/oder eine Steckverbindung, umfasst.

3. Sensor (1) nach Anspruch 1 oder 2, **dadurch gekennzeichnet, dass** ein Gummizug (4)
- an der Gummizug-Halterung (16) des Sensors (1) reversibel befestigt werden kann um die Gummizug-Halterung (16) mit einem Bracket (23), Bogendraht (24) oder Aligner (2', 3') zu verbinden, wobei gegebenenfalls die Gummizug-Halterung (16) bei gespanntem Gummizug (4) eine Kraft auf den Druck-, Zug- und/oder Scherkraftsensor (11) des Sensors (1) ausübt, und/oder
- in der Gummizug-Führung (17) des Sensors (1) geführt werden kann, wodurch eine Kraft auf den Druck-, Zug- und/oder Scherkraftsensor (11) ausgeübt wird, wenn der Gummizug (4) von einem Bracket (23) der Multibracket-Apparatur des Oberkiefers (2) oder vom Aligner des Oberkiefer (2') zu einem Bracket (23) der Multibracket-Apparatur des Unterkiefers (3) oder zum Aligner des Unterkiefers (3') angeordnet ist, wobei gegebenenfalls der Gummizug (4) anstelle an einem Bracket (23) oder Aligner (2', 3') auch an der Gummizug-Halterung (16) des Sensors (1) befestigt sein kann.

4. Sensor (1) nach mindestens einem der Ansprüche 1 bis 3, **dadurch gekennzeichnet, dass** die Befestigungsvorrichtung (15) eine Öse, Klemme, Schraube, ein Draht, Gummizug, Steckverbindung, Elastik und/oder Klebefläche umfasst, anhand welcher der Sensor (1) im Wesentlichen an jede beliebige Multibracket-Apparatur (2, 3) und an jeden beliebigen Aligner (2', 3') befestigt werden kann.

5. System (5) zur Überwachung der Tragedauer von kieferorthopädischen Gummizügen für Multibracket-Apparaturen (2, 3) und/oder Aligner (2', 3'), umfassend einen Sensor (1) nach mindestens einem der Ansprüche 1 bis 4, eine Multibracket-Apparatur oder einen Aligner für den Oberkiefer (2, 2') und eine Multibracket-Apparatur oder einen Aligner für den Unterkiefer (3, 3'), sowie einen Gummizug (4) und ein externes Lesegerät, wobei die Multibracket-Apparaturen (2, 3) eine Vielzahl von Brackets (23) und je einen Bogendraht (24) umfassen.

6. System (5) nach Anspruch 5, **dadurch gekennzeichnet, dass** die Befestigungsvorrichtung (15) des Sensors (1) nach mindestens einem der Ansprüche 1 bis 4, mit mindestens einem Bracket (23) und/oder dem Bogendraht (24) einer Multibracket-Apparatur (2, 3), und/oder einem Aligner (2', 3'), mit einem Befestigungsmittel verbunden werden kann.

7. System (5) nach Anspruch 6, **dadurch gekennzeichnet, dass** das Befestigungsmittel einen Draht, eine Schraube, eine Klemme, einen Gummizug, einen Elastik, einen Kunststoff und/oder einen Klebstoff oder eine Klebstoffkombination umfasst.

8. System (5) nach mindestens einem der Ansprüche 5 bis 7, **dadurch gekennzeichnet, dass** der Sensor (1) nach mindestens einem der Ansprüche 1 bis 4, wenn er an einer Multibracket-Apparatur (2, 3) befestigt ist,
- zwischen zwei Brackets (23) der gleichen Multibracket-Apparatur (2, 3) angeordnet ist, und/oder
- sich über mindestens ein, bevorzugt über mindestens zwei, Brackets (23) der gleichen Multibracket-Apparatur (2, 3) erstreckt.

9. System (5) nach mindestens einem der Ansprüche 5 bis 7, **dadurch gekennzeichnet, dass** der Sensor (1) nach mindestens einem der Ansprüche 1 bis 4, wenn er an einem Aligner (2', 3') befestigt ist,
- im seitlichen Aussenbereich des Aligners (2', 3') angeordnet ist, und/oder
- sich über mindestens einen, bevorzugt über mindestens zwei, Zahnkronen des gleichen Aligners (2', 3') erstreckt.

10. Verfahren zum Überwachen der Tragedauer von kieferorthopädischen Gummizügen an Multibracket-Apparaturen (2, 3) und/oder Aligner (2', 3') mit dem System (5) nach mindestens einem der Ansprüche 5 bis 9, **dadurch gekennzeichnet, dass**
v) ein Sensor (1) nach mindestens einem der Ansprüche 1 bis 4 an einer Multibracket-Apparatur (2, 3) und/oder an einem Aligner (2', 3') befestigt wird,
vi) der Gummizug (4) von einer Multibracket-Apparatur (2, 3) und/oder von einem Aligner (2', 3') zur anderen Multibracket-Apparatur (2, 3) und/oder Aligner (2', 3') gespannt wird, wobei der Gummizug (4) an der Gummizug-Halterung (16) befestigt wird und/oder der Gummizug (4) in der Gummizug-Führung (17) geführt wird, wodurch durch den Gummizug (4) eine Kraft und/oder eine Scherkraft auf den Druck-, Zug- und/oder Scherkraftsensor (11) des Sensors (1) ausgeübt wird,
vii) mindestens die Zeitspanne der auf den Druck-, Zug- und/oder Scherkraftsensor (11) ausgeübten Kraft und/oder Scherkraft gemessen und auf dem Datenspeicher (12) gespeichert wird, und
viii) die im Datenspeicher (12) gespeicherten Daten unter Verwendung des Lesegeräts ausgelesen werden.

11. Verfahren nach Anspruch 11, **dadurch gekennzeichnet, dass** die im Datenspeicher (12) gespeicherten Daten mittels Funk, insbesondere mittels NFC, RFID und/oder Bluetooth, und/oder mittels Steckverbindung zum externen Lesegerät übertragen und gegebenenfalls gespeichert und/oder ausgedruckt werden.

12. Verwendung des Sensors (1) nach mindestens einem der Ansprüche 1 bis 4, des Systems (5) nach mindestens einem der Ansprüche 5 bis 9 und des Verfahrens nach Anspruch 10 oder 11 zum Überwachen der Tragedauer von kieferorthopädischen Gummizügen an Multibracket-Apparaturen (2, 3) und Alignern (2', 3') und/oder zur Messung der durch den Gummizug (4) ausgeübten Kraft, insbesondere der ausgeübten Kraft in Funktion der Tragedauer.
